# FASCICULE DE BREVET EUROPEEN

(11) **EP 2 118 104 B1**
(45) Date de publication et mention de la délivrance du brevet: **02.01.2013**
(21) Numéro de dépôt: 08761840.1
(22) Date de dépôt: 04.02.2008
(51) Int. Cl.: C07D 471/08, C07D 487/08, A61K 31/4748, A61P 25/00, A61P 29/00, A61P 9/00

(54) **DÉRIVÉS D'AZABICYCLOALKANE, LEUR PRÉPARATION ET LEUR APPLICATION EN THÉRAPEUTIQUE**
AZABICYCLOALKAN-DERIVATE, HERSTELLUNG DAVON UND THERAPEUTISCHE ANWENDUNG
AZABICYCLOALKANE DERIVATIVES, PREPARATION THEREOF AND USE THEREOF IN THERAPY

(30) Priorité: 09.02.2007 FR 0700940
(43) Date de publication de la demande: 18.11.2009
(73) Titulaire: SANOFI, 75008 Paris (FR)
(72) Inventeur: BEN AYAD, Omar, F-75013 Paris (FR); LECLERC, Odile, F-75013 Paris (FR); LOCHEAD, Alistair, F-75013 Paris (FR); SAADY, Mourad, F-75013 Paris (FR); SLOWINSKI, Franck, F-75013 Paris (FR); VACHE, Julien, F-75013 Paris (FR)
(74) Mandataire: Kugel, Dominique
(86) Numéro de dépôt international: PCT/FR2008/000137
(87) Numéro de publication internationale: WO 2008/110699

(56) Documents cités:
- WO-A-03/057697
- WO-A-2007/020344

## Description

La présente invention se rapporte à des dérivés d'azabicycloalkane, à leur préparation et à leur application en thérapeutique.

Le document WO03/057697A décrit des dérivés de 5-(pyridin-3-yl)-1-azabicyclo[3.2.1]octane ligands des récepteurs nicotiniques, et qui sont utiles dans le traitement ou la prévention des désordres liés à un dysfonctionnement des récepteurs nicotiniques, notamment au niveau du système nerveux central.
Il existe toujours une nécessité de trouver et de développer des produits affins pour les récepteurs nicotiniques.
L'invention répond à ce but en proposant des composés nouveaux, qui présentent une affinité pour les récepteurs nicotiniques.

La présente invention a pour objet les composés répondant à la formule générale (I) 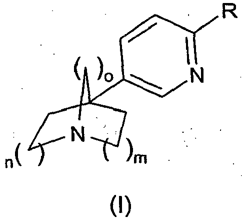 dans laquelle :
R représente
soit un atome d'hydrogène ou d'halogène ;
soit un groupe : hydroxyle, (C₁-C₆)alcoxy, (C₃-C₇)cycloalkyle-O- ou (C₃-C₇)cycloalkyle-(C₁-C₃)alkylène-O- ;
soit un groupe hétérocycloalkyle, aryle ou hétéroaryle ; ce groupe pouvant éventuellement être substitué par un ou plusieurs groupes choisis parmi les atomes d'halogènes, les groupes (C₁-C₆)alkyle, (C₃-C₇)cycloalkyle, (C₃-C₇)cycloalkyle-(C₁-C₃)alkylène, (C₁-C₆)alcoxy, (C₃-C₇)cycloalkyle-O-, (C₃-C₇)cycloalkyle-(C₁-C₃)alkylène-O-, (C₁-C₆)fluoroalkyle, (C₁-C₆)fluoroalcoxy, nitro, cyano, hydroxyle, amino, (C₁-C₆)alkylamino ou di(C₁-C₆)alkylamino, hétérocycloalkyle, aryle, aryle-(C₁-C₆)alkylène, hétéroaryle, hétéroaryle-(C₁-C₆)alkylène, aryle-O-, -C(O)-(C₁-C₆)alkyle,
le groupe hétérocycloalkyle étant éventuellement substitué par-C(O)O(CH₃)₃ ;
n représente 1 ou 2 ;
m représente 1 ou 2 ;
o représente 1 ou 2 ;
le cas où n et o =1 et m = 2 étant exclu ;
les composés suivants étant exclus :
   - 4-(6-fluoropyridin-3-yl)-1-azabicyclo[2.2.1]heptane ;
   - 4-(6-chloropyridin-3-yl)-1-azabicyclo[2.2.1]heptane ;
   - 4-(6-bromopyridin-3-yl)-1-azabicyclo[2.2.1]heptane ;
   - 4-(pyridin-3-yl)-1-azabicyclo[2.2.1]heptane.

Le 4-(6-fluoropyridin-3-yl)-1-azabicyclo[2.2.1]heptane, le 4-(6-chloropyridin-3-yl)-1-azabicyclo[2.2.1]heptane, le 4-(6-bromopyridin-3-yl)-1-azabicyclo[2.2.1]heptane et le 4-(pyridin-3-yl)-1-azabicyclo[2.2.1]heptane sont décrits dans le document WO95/03306A comme composés arthropodicides.

Les composés de formule (I) peuvent comporter un ou plusieurs centres stéréogènes, tel que par exemple un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent également exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- Cₜ-C_{z} où t et z peuvent prendre les valeurs de 1 à 7 : une chaîne carbonée pouvant avoir de t à z atomes de carbone, par exemple C₁-C₃ une chaîne carbonée qui peut avoir de 1 à 3 atomes de carbone ;
- un atome d'halogène : un atome de fluor, de chlore, de brome ou d'iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe alcoxy : un radical -O-alkyle dont le groupe alkyle est tel que précédemment défini ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un fluoroalcoxy : un groupe alcoxy dont un ou plusieurs atomes d'hydrogène ont été substitués par un atome de fluor ;
- un groupe hétérocycloalkyle : un groupe cyclique de 3 à 7 chaînons contenant de 1 à 2 hétéroatomes choisis parmi O, S ou N ; A titre d'exemples de groupes hétérocycloalkyles, on peut citer les groupes pyrrolidinyle, pipérazinyle, morpholinyle ;
- un groupe aryle : un groupe aromatique cyclique comprenant entre 6 et 10 atomes de carbones. A titre d'exemples de groupes aryles, on peut citer les groupes phényle ou naphtyle ;
- un groupe hétéroaryle : un groupe cyclique partiellement saturé ou aromatique de 5 à 15 chaînons contenant de 1 à 4 hétéroatomes choisis parmi O, S ou N. A titre d'exemple, on peut citer les groupes indolyle, furyle, pyrrolyle, thiazolyle, pyrazolyle, pyridinyle, pyrimidinyle, benzodioxolyle, benzofuryle, benzothiènyle, benzoxadiazolyle, benzotriazolyle, quinolinyle, isoquinolinyle, dibenzofuryle, dibenzothiènyle, imidazolyle, triazolyle, tétrazolyle, oxazolyle, oxadiazolyle, thiadiazolyle, thiényle, isoxazolyle, isothiazolyle.

Parmi les composés de formule (I) objets de l'invention, un premier sous-groupe de composés est constitué par les composés pour lesquels :
R représente
soit un atome d'hydrogène ou d'halogène ;
soit un groupe hydroxyle ;
soit un groupe hétérocycloalkyle, aryle ou hétéroaryle ; ce groupe pouvant éventuellement être substitué par un ou plusieurs groupes choisis parmi les atomes d'halogènes, les groupes (C₁-C₆)alkyle, (C₁-C₆)alcoxy, (C₁-C₆)fluoroalkyle, (C₁-C₆)fluoroalcoxy, nitro, cyano, hydroxyle, amino, (C₁-C₆)alkylamino ou di(C₁-C₆)alkylamino, hétérocycloalkyle, aryle, aryle-(C₁-C₆)alkylène, hétéroaryle, aryle-O-, -C(O)-(C₁-C₆)alkyle, le groupe hétérocycloalkyle étant éventuellement substitué par -C(O)O(CH₃)₃ ;
n représente 1 ou 2 ;
m représente 1 ou 2 ;
o représente 1 ou 2.

Parmi les composés de formule (I) objets de l'invention, un second sous-groupe de composés est constitué par les composés pour lesquels :
R représente
soit un atome d'halogène, plus particulièrement un chlore ;
soit un groupe hydroxyle ;
soit un groupe hétérocycloalkyle, plus particulièrement un groupe pyrrolidinyle ou pipérazinyle, soit un groupe aryle, plus particulièrement un groupe phényle ou naphtyle, soit un groupe hétéroaryle, plus particulièrement un groupe pyrazolyle, indolyle, pyridinyle, benzofuryle, quinolinyle, benzothiènyle, furyle, dibenzofuryle, pyrrolyle, pyrimidinyle, benzotriazolyle, dibenzothiènyle, benzoxadiazolyle, thiazolyle ou isoquinolinyle ; ce groupe pouvant éventuellement être substitué par un ou plusieurs groupes, plus particulièrement par un ou deux groupes, choisis parmi les atomes d'halogènes, plus particulièrement le fluor, les groupes (C₁-C₆)alkyle, plus particulièrement méthyle, (C₁-C₆)alcoxy, plus particulièrement méthoxy, (C₁-C₆)fluoroalkyle, plus particulièrement trifluorométhyle, (C₁-C₆)fluoroalcoxy, plus particulièrement trifluorométhoxy, di(C₁-C₆)alkylamino, plus particulièrement diméthylamino, hétérocycloalkyle, plus particulièrement morpholinyle, pyrrolidinyle ou pipérazinyle, aryle, plus particulièrement phényle, aryle-(C₁-C₆)alkylène, plus particulièrement benzyle, hétéroaryle, plus particulièrement pyrazolyle, aryle-O-, plus particulièrement phénoxy, -C(O)-(C₁-C₆)alkyle, plus particulièrement -C(O)-CH₃ ; le groupe hétérocycloalkyle étant éventuellement substitué par - C(O)O(CH₃)₃ ;
n représente 1 ou 2 ;
m représente 1 ou 2 ;
o représente 1.

Parmi les composés de formule (I) objets de l'invention et du second sous-groupe ci-dessus, un troisième sous-groupe de composés est constitué par les composés pour lesquels :
n et m et o = 1.

Parmi les composés de formule (I) objets de l'invention et du second sous-groupe ci-dessus, un quatrième sous-groupe de composés est constitué par les composés pour lesquels :
n et m = 2 et o = 1.

Parmi les composés de formule (I) objets de l'invention, on peut notamment citer les composés suivants :
**1.** 4-[6-(1-méthyl-1*H*-pyrazol-4-yl)pyridin-3-yl]-1-azabicyclo[2.2.1]heptane
**2.** 5-[6-(1-méthyl-1*H*-pyrazol-4-yl)pyridin-3-yl]-1-azabicyclo[3.3.1]nonane
**3.** 5-[6-(3,4-diméthoxyphényl)pyridin-3-yl]-1-azabicyclo[3.3.1]]nonane
**4.** 5-(1-azabicyclo[3.3.1]non-5-yl)pyridin-2-ol
**5.** 5-(6-chloropyridin-3-yl)-1-azabicyclo[3.3.1]nonane
**6.** 5-[5-(1-azabicyclo[2.2.1]hept-4-yl)pyridin-2-yl]-1-méthyl-1*H*-indole
**7.** 5-(1-azabicyclo[3.3.1]non-5-yl)-2,4'-bipyridine
**8**. 4-[6-(4-fluorophényl)pyridin-3-yl]-1-azabicyclo[2.2.1]heptane
**9.** 4-[6-(1-benzofur-2-yl)pyridin-3-yl]-1-azabicyclo[2.2.1]heptane
**10.** 5-[6-(1-benzofur-2-yl)pyridin-3-yl]-1-azabicyclo[3.3.1]nonane
**11.** 5-(1-azabicyclo[2.2.1]hept-4-yl)-2,4'-bipyridine
**12.** 6-[5-(1-azabicyclo[2.2.1]hept-4-yl)pyridin-2-yl]quinoline
**13.** 4-[6-(4-benzylpipérazin-1-yl)pyridin-3-yl]-1-azabicyclo[2.2.1]heptane
**14.** 4-[6-(1-benzothièn-2-yl)pyridin-3-yl]-1-azabicyclo[2.2.1]heptane
**15.** 5-(1-azabicyclo[2.2.1]hept-4-yl)pyridin-2-ol
**16.** 4-[6-(1,3-benzodioxol-5-yl)pyridin-3-yl]-1-azabicyclo[2.2.1]heptane
**17.** 4-[6-(3,4-diméthoxyphényl)pyridin-3-yl]-1-azabicydo[2.2.1]heptane
**18.** 4-{6-[3-(trifluorométhyl)phényl]pyridin-3-yl}-1-azabicyclo[2.2.1]heptane
**19.** 4-[6-(3-fluorophényl)pyridin-3-yl]-1-azabicyclo[2.2.1]heptane
**20.** 5-[6-(3-fluorophényl)pyridin-3-yl]-1-azabicyclo[3.3.1]nonane
**21.** 4-[6-(3-furyl)pyridin-3-yl]-1-azabicyclo[2.2.1]heptane
**22.** 4-(6-dibenzo[*b*,*d*]fur-4-ylpyridin-3-yl)-1-azabicyclo[2.2.1]heptane
**23.** 3-[5-(1-azabicyclo[2.2.1]hept-4-yl)pyridin-2-yl]quinoline
**24.** 5-(1-azabicyclo[2.2.1]hept-4-yl)-2,3'-bipyridine
**25.** 4-{6-[4-(triftuorométhoxy)phényl]pyridin-3-y}-1-azabicyclo[2.2.1]heptane
**26.** 5-[6-(1-benzothièn-2-yl)pyridin-3-yl]-1-azabicyclo[3.3.1]nonane
**27.** 4-[6-(4-fluoro-2-méthoxyphényl)pyridin-3-yl]-1-azabicyclo[2.2.1]heptane
**28.** 4-[6-(1*H*-pyrrol-3-yl)pyridin-3-yl]-1-azabicyclo[2.2.1]heptane
**29.** 4-(6-pyrrolidin-1-ylpyridin-3-yl)-1-azabicyclo[2.2.1]heptane
**30.** 5-(6-dibenzo[*b*,*d*]fur-4-ylpyridin-3-yl)-1-azabicyclo[3.3.1]nonane
**31.** 5-[6-(1,3-benzodioxol-5-yl)pyridin-3-yl]-1-azabicyclo[3.3.1]nonane
**32.** 6-[5-(1-azabicyclo[3.3.1]non-5-yl)pyridin-2-yl]quinoline
**33.** 5-[5-(1-azabicyclo[3.3.1]non-5-yl)pyridin-2-yl]-1-méthyl-1*H*-indole
**34.** 5-[6-(1*H*-pyrrol-3-yl)pyridin-3-yl]-1-azabicyclo[3.3.1]nonane
**35.** 5-{6-[3-(trifluorométhyl)phényl]pyridin-3-yl}-1-azabicyclo[3.3.1]nonane
**36.** 5-[6-(4-fluorophényl)pyridin-3-yl]-1-azabicyclo[3.3.1]nonane
**37.** 4-(6-pyrimidin-5-ylpyridin-3-yl)-1-azabicyclo[2.2.1]heptane
**38.** 4-[6-(2,4-diméthoxypyrimidin-5-yl)pyridin-3-yl]-1-azabicyclo[2.2.1]heptane
**39.** 5-(6-biphényl-4-ylpyridin-3-yl)-1-azabicydo[3.3.1]nonane
**40.** 5-[6-(3-furyl)pyridin-3-yl]-1-azabicyclo[3.3.1]nonane
**41.** 5-(1-azabicyclo[3.3.1]non-5-yl)-2,3'-bipyridine
**42.** 5-{6-[4-(trifluorométhoxy)phényl]pyridin-3-yl}-1-azabicyclo[3.3.1]nonane
**43.** 5-[6-(4-fluoro-2-méthoxyphényl)pyridin-3-yl]-1-azabicydo[3.3.1]nonane
**44.** 3-[5-(1-azabicyclo[3.3.1]non-5-yl)pyridin-2-yl]quinoline
**45.** 4-(6-biphényl-4-ylpyridin-3-yl)-1-azabicyclo[2.2.1]]heptane
**46.** 8-[5-(1-azabicyclo[2.2.1]hept-4-yl)pyridin-2-yl]quinoline
**47.** 1-[5-(1-azabicyclo[3.3.1]non-5-yl)pyridin-2-yl]-1*H*-1,2,3-benzotriazole
**48.** 5-(6-dibenzo[*b*,*d*]thièn-2-ylpyridin-3-yl)-1-azabicyclo[3.3.1]nonane
**49.** 5-[6-(4-méthoxyphényl)pyridin-3-yl]-1-azabicyclo[3.3.1]nonane
**50.** 5-[6-(3-méthoxyphényl)pyridin-3-yl]-1-azabicyclo[3.3.1]nonane
**51.** 5-[5-(1-azabicyclo[2.2.1]hept-4-yl)pyridin-2-yl]-2,1,3-benzoxadiazole
**52.** 4-[6-(2-morpholin-4-ylpyrimidin-5-yl)pyridin-3-yl]-1-azabicyclo[2.2.1]heptane
**53.** 4-[6-(1-méthyl-1*H*-pyrrol-2-yl)pyridin-3-yl]-1-azabicyclo[2.2.1]heptane
**54.** 4-[6-(3-fluoro-5-méthoxyphényl)pyridin-3-yl]-1-azabicyclo[2.2.1]heptane
**55.** 4-[6-(3-fluoro-4-méthoxyphényl)pyridin-3-yl]-1-azabicyclo[2.2.1]heptane
**56.** 4-[6-(2-fluoro-3-méthoxyphényl)pyridin-3-yl]-1-azabicyclo[2.2.1]heptane
**57.** 5-[6-(3-fluoro-4-méthoxyphényl)pyridin-3-yl]-1-azabicyclo[3.3.1]nonane
**58.** 5-[6-(2-fluoro-3-méthoxyphényl)pyridin-3-yl]-1-azabicyclo[3.3.1]nonane
**59.** 5-[6-(1-méthyl-1*H*-pyrrol-2-yl)pyridin-3-yl]-1-azabicyclo[3.3.1]nonane
**60.** 5-[6-(2-furyl)pyridin-3-yl]-1-azabicyclo[3.3.1]nonane
**61.** 5-{6-[3-(1*H*-pyrazol-1-yl)phényl]pyridin-3-yl}-1-azabicyclo[3.3.1]nonane
**62.** 4-[6-(thiazol-2-yl)pyridin-3-yl]-1-azabicyclo[2.2.1]heptane
**63.** 5-(6-pyrimidin-5-ylpyridin-3-yl)-1-azabicyclo[3.3.1]nonane
**64.** 5-[6-(1-naphthyl)pyridin-3-yl]-1-azabicyclo[3.3.1]nonane
**65.** 5-[6-(2-morpholin-4-ylpyrimidin-5-yl)pyridin-3-yl]-1-azabicyclo[3.3.1]nonane
**66.** 5-[6-(2,4-diméthoxypyrimidin-5-yl)pyridin-3-yl]-1-azabicyclo[3.3.1]nonane
**67.** 5-[5-(1-azabicyclo[3.3.1]non-5-yl)pyridin-2-yl]-2,1,3-benzoxadiazole
**68.** 5-(6-phénylpyridin-3-yl)-1-azabicyclo[3.3.1]nonane
**69.** 5-[6-(2,5-diméthoxyphényl)pyridin-3-yl]-1-azabicyclo[3.3.1]nonane
**70.** 5-{6-[4-(trifluorométhyl)phényl]pyridin-3-yl}-1-azabicyclo[3.3.1]nonane
**71.** 5-(1-azabicyclo[3.3.1]non-5-yl)-4'-méthoxy-2,3'-bipyridine
**72.** 4-[5-(1-azabicyclo[3.3.1]non-5-yl)pyridin-2-yl]isoquinoline
**73.** 5-{6-[3,5-bis(trifluorométhyl)phényl]pyridin-3-yl}-1-azabicyclo[3.3.1]nonane
**74.** 5-[6-(thiazol-2-yl)pyridin-3-yl]-1-azabicyclo[3.3.1]nonane
**75.** 5-[6-(4-phénoxyphényl)pyridin-3-yl]-1-azabicyclo[3.3.1]nonane
**76.** 5-[6-(2-fluorophényl)pyridin-3-yl]-1-azabicyclo[3.3.1]nonane
**77.** 5-(1-azabicyclo[3.3.1]non-5-yl)-6'-pyrrolidin-1-yl-2,3'-bipyridine
**78.** 4-[5-(1-azabicyclo[3.3.1]non-5-yl)-2,3'-bipyridin-6'-yl]pipérazine-1-carboxylate de *tert*-butyle
**79.** 5-[6-(2-méthoxyphényl)pyridin-3-yl]-1-azabicyclo[3.3.1]nonane
**80.** 5-[6-(2,4-diméthoxyphényl)pyridin-3-yl]-1-azabicyclo[3.3.1]nonane
**81.** 5-{6-[3-(trifluorométhoxy)phényl]pyridin-3-yl}-1-azabicyclo[3.3.1]nonane
**82.** 3-[5-(1-azabicyclo[3.3.1]non-5-yl)pyridin-2-yl]-*N*,*N*-diméthylaminophényle
**83.** 4-[5-(1-azabicyclo[2.2.1]hept-4-yl)pyridin-2-yl]isoquinoline
**84.** 5-[5-(1-azabicyclo[2.2.1]hept-4-yl)pyridin-2-yl]isoquinoline
**85.** 4-(6-phénylpyridin-3-yl)-1-azabicyclo[2.2.1]heptane
**86.** 4-{6-[3,5-bis(trifluorométhyl)phényl]pyridin-3-yl}-1-azabicyclo[2.2.1]heptane
**87.** 5-(1-azabicyclo[2.2.1]hept-4-yl)-4'-méthoxy-2,3'-bipyridine
**88.** 4-[5-(1-azabicyclo[2.2.1]hept-4-yl)-2,3'-bipyridin-6'-yl]pipérazine-1-carboxylate de *tert*-butyle
**89.** 5-[5-(1-azabicyclo[3.3.1]non-5-yl)pyridin-2-yl]isoquinoline
**90.** 5-(1-azabicyclo[2.2.1]hept-4-yl)-6'-fluoro-2,3'-bipyridine
**91.** 5-(1-azabicyclo[3.3.1]non-5-yl)-2'-fluoro-2,3'-bipyridine
**92.** 4-[6-(2-méthoxyphényl)pyridin-3-yl]-1-azabicyclo[2.2.1]heptane
**93.** 4-[6-(2,4-diméthoxyphényl)pyridin-3-yl]-1-azabicyclo[2.2.1]heptane
**94.** 4-[6-(2,5-diméthoxyphényl)pyridin-3-yl]-1-azabicyclo[2.2.1]heptane
**95.** 4-[6-(2-fluorophényl)pyridin-3-yl]-1-azabicyclo[2.2.1]heptane
**96.** 4-[6-(4-méthoxyphényl)pyridin-3-yl]-1-azabicyclo[2.2.1]heptane
**97.** 4-[6-(2-naphthyl)pyridin-3-yl]-1-azabicyclo[2.2.1]heptane
**98.** 4-[6-(4-phénoxyphényl)pyridin-3-yl]-1-azabicyclo[2.2.1]heptane
**99.** 4-{6-[2-(trifluorométhyl)phényl]pyridin-3-yl}-1-azabicyclo[2.2.1]heptane
**100.** 5-[6-(2-naphthyl)pyridin-3-yl]-1-azabicyclo[3.3.1]nonane
**101.** 5-(1-azabicyclo[2.2.1]hept-4-yl)-2'-fluoro-2,3'-bipyridine
**102.** 5-(1-azabicyclo[3.3.1]non-5-yl)-6'-fluoro-2,3'-bipyridine
**103.** 4-[6-(3-Trifluorométhoxy-phényl)-pyridin-3-yl]-1-aza-bicyclo[2.2.1]heptane
**104.** 5-[5-(1-Aza-bicyclo[3.3.1]non-5-yl)-pyridin-2-yl]-quinoline
**105.** 5-[6-(2-Fluoro-biphényl-4-yl)-pyridin-3-yl]-1-aza-bicyclo[3.3.1]nonane
**106.** 1-{3-[5-(1-Aza-bicyclo[3.3.1]non-5-yl)-pyridin-2-yl]-phényl}-éthanone
**107.** 1-{3-[5-(1-Aza-bicyclo[2.2.1]hept-4-yl)-pyridin-2-yl]-phényl}-ethanone
**108.** 4-(6-(4-méthylphényl)-pyridin-3-yl)-1-aza-bicyclo[2.2.1]heptane
**109.** 4-[6-(2-Fluoro-biphényl-4-yl)-pyridin-3-yl]-1-aza-bicyclo[2.2.1]heptane
**110.** 5-[5-(1-Aza-bicyclo[2.2.1]hept-4-yl)-pyridin-2-yl]-quinoline
**111.** 4-[6-(3-Méthoxy-phényl)-pyridin-3-yl]-1-aza-bicyclo[2.2.1]heptane
**112.** 4-[6-(3,4-Difluoro-phényl)-pyridin-3-yl]-1-aza-bicyclo[2.2.1]heptane
**113.** 4-[6-(3-Pyrazol-1-yl-phényl)-pyridin-3-yl]-1-aza-bicyclo[2.2.1]heptane

Dans ce qui suit, on entend par groupe protecteur un groupe qui permet, d'une part, de protéger une fonction réactive telle qu'un hydroxyle ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Conformément à l'invention et dans le cas où n et m et o = 1, on peut préparer les composés de formule générale (I) par le procédé illustré par le schéma 1 qui suit. On fait réagir le composé de formule (II), avec le bromure d'acétate d'éthyle de formule générale (III), dans laquelle R₁ représente un groupe (C₁-C₄)alkyle, en présence d'une base telle que le diisopropylamidure de lithium, pour obtenir un composé de formule générale (IV). L'hydrogénation de la fonction nitrile sous atmosphère d'hydrogène comprise entre 1 et 6 atmosphères en présence d'un catalyseur tel que par exemple le Nickel de Raney, fournit un composé de formule générale (V). Le composé de formule générale (V) est réduit par un réducteur tel que par exemple l'hydrure mixte d'aluminium et de lithium pour conduire au composé de formule (VI). Le composé de formule (VI) est transformé en composé de formule (VII), par exemple en présence d'acide bromhydrique concentré. Le composé de formule (VII) est mis en réaction en milieu basique avec une base telle que le carbonate de sodium pour obtenir un composé de formule (VIII). Le traitement de ce composé, par exemple par de l'oxychlorure de phosphore, conduit au composé de formule (IX). Les composés de formule générale (I) peuvent ensuite être préparés à partir du composé de formule (IX) selon toutes méthodes connues de l'homme du métier, telles que par exemple :
- avec un acide boronique de formule R-B(OH)₂ dans laquelle R est tel que défini dans la formule générale (I), en présence d'un catalyseur au palladium, par exemple le tétrakistriphénylphosphine palladium ;
- avec un composé de formule R-H dans laquelle R est tel que défini dans la formule générale (I), en présence ou non d'une base forte, par exemple l'hydrure de sodium, dans un solvant, par exemple le diméthylformamide ou sans solvant ;
- avec un composé de formule R-H dans laquelle R est tel que défini dans la formule générale (I), en présence d'une base, par exemple le *tert*-butanolate de sodium, d'un catalyseur au palladium, par exemple le palladium(II) acétate et d'un ligand, par exemple le 2-dicyclohexylphosphino-2'-(N,N-diméthylamino)biphényl ;

- avec un dérivé stanneux de formule R-Sn[(CH₂)₃CH₃)]₃ dans laquelle R est tel que défini dans la formule générale (I), en présence d'un catalyseur au palladium, par exemple le bis(triphénylphosphino)dichloropalladium ;
- avec un composé de formule R-H dans laquelle R est tel que défini dans la formule générale (I), en présence de n-butyllithium, de chlorure de zinc et d'un catalyseur au palladium, par exemple le tétrakistriphénylphosphine palladium.

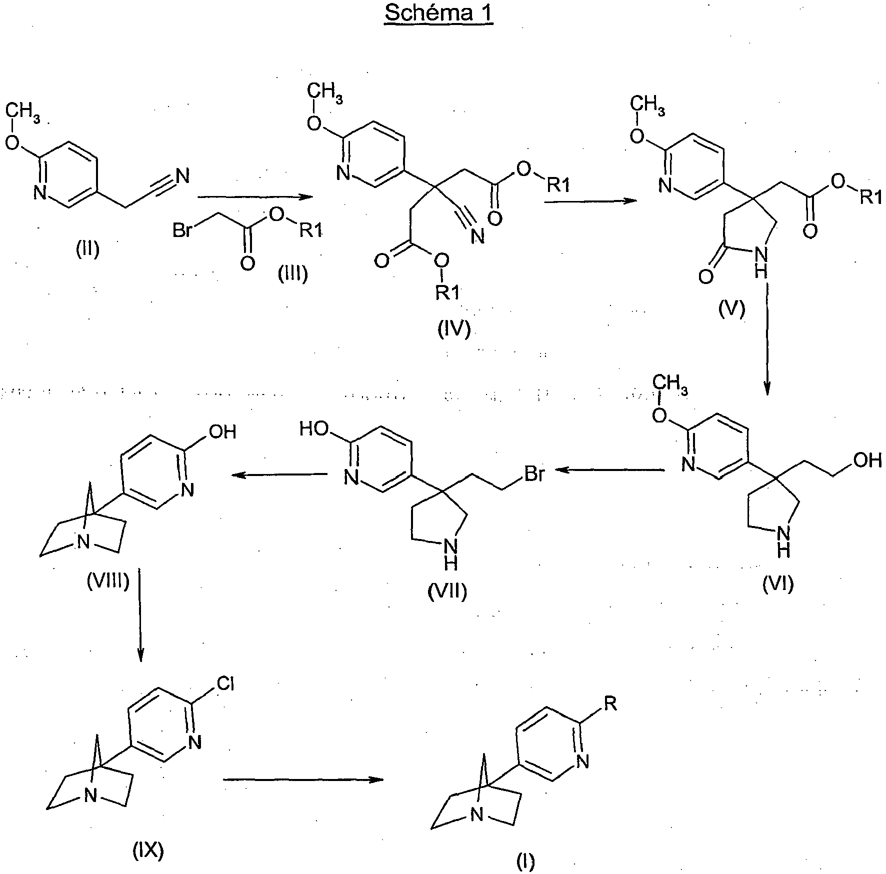

Conformément à l'invention et dans le cas où n et m = 2 et o = 1, on peut préparer les composés de formule générale (I) par le procédé illustré par le schéma 2 qui suit.
On fait réagir le composé de formule (II), avec l'acrylate d'éthyle de formule générale (X), dans laquelle R₁ représente un groupe (C₁-C₄)alkyle, en présence d'une base telle que le triton B, pour obtenir un composé de formule générale (XI). Le composé de formule générale (I) est ensuite obtenu à partir des intermédiaires (XII) à (XVI) selon des étapes analogues à celles ayant permis de préparer les intermédiaires (V) à (IX) comme décrit précédemment dans le schéma1.

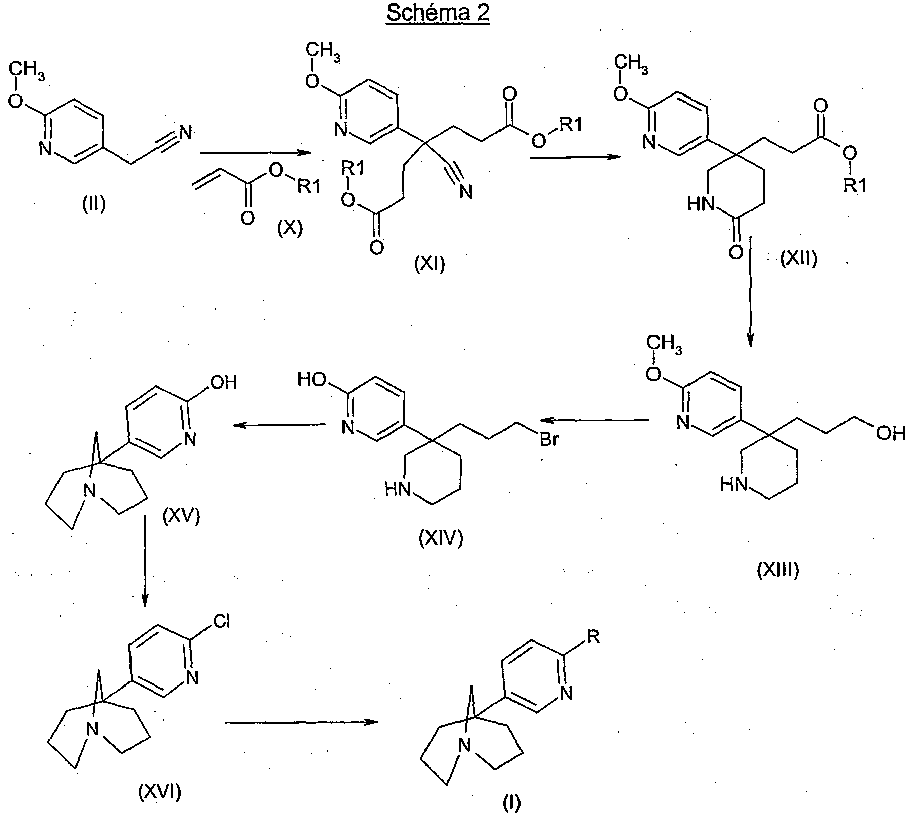

Le composé de formule (II) est accessible par des méthodes décrites dans la littérature, comme par exemple dans le document WO2004/111031.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les composés de formule générale (I) correspondants aux autres valeurs de n, m et o peuvent être obtenus par adaptation des procédés décrits dans les schémas 1 et 2 selon des méthodes classiques de synthèse de chimie organique adaptables par l'homme du métier.

L'invention, selon un autre de ses aspects, a également pour objet les composés de formules (III) à (XVI). Ces composés sont utiles comme intermédiaires de synthèse des composés de formule générale (I).
Notamment, l'invention a pour objet l'intermédiaire (XVI).

Les exemples suivants décrivent la préparation de certains composés conformes à l'invention. Ces exemples ne sont pas limitatifs et ne font qu'illustrer la présente invention. Les numéros des composés donnés entre parenthèses dans les titres renvoient à ceux donnés dans la première colonne du tableau ci-après, qui illustre les structures chimiques et les propriétés physiques de quelques composés selon l'invention.

### Exemple 1 (Composé N°4)

### 5-(1-azabicyclo[3.3.1]non-5-yl)pyridin-2-ol

### 1.1 4-cyano-4-(6-méthoxypyridin-3-yl)heptanedioate de diéthyle

Dans un ballon de 250 ml on introduit 2,775g (18,73 mmoles) de (6-méthoxy-pyridin-3-yl)-acétonitrile (WO2004/111031) dans 125 ml d'acétonitrile anhydre et sous atmosphère d'argon. On additionne 0,87 ml (1,87 mmole) de triton B (40% dans le méthanol), le mélange réactionnel est porté à reflux puis 20,40 ml (187,30 mmoles) d'acrylate d'éthyle sont additionnés au goutte à goutte. Le milieu réactionnel est ensuite agité à reflux pendant 48 heures, refroidit à température ambiante et concentré sous pression réduite. Le résidu ainsi obtenu est versé dans une solution aqueuse saturée en chlorure d'ammonium, extrait deux fois par du dichlorométhane. Les phases organiques sont réunies, séchées sur sulfate de sodium, filtrées et concentrées sous pression réduite. On purifie le résidu obtenu par chromatographie sur colonne de gel de silice en éluant par un mélange de chloroforme, méthanol et ammoniaque dans les proportions 90/10/1.
On obtient 7,981 g de produit sous forme d'une huile orange.
RMN ¹H (CDCl₃ ; 200 MHz) : δ (ppm): 8,18 (d, 1H) ; 7,45 (dd, 1H) ; 6,71 (d, 1H) ; 4,00 (q, 4H) ; 3,87 (s, 3H) ; 2,55-1,97 (m, 8H) ; 1,14 (t, 6H).

### 1.2 3-[3-(6-méthoxypyridin-3-yl)-6-oxopipéridin-3-yl]propanoate d'éthyle

Dans un flacon d'hydrogénation on introduit 1,999 g (5,74 mmoles) de 4-cyano-4-(6-méthoxypyridin-3-yl)heptanedioate de diéthyle, tel que obtenu à l'étape 1.1, en solution dans 115 ml d'alcool éthylique, en présence de Nickel de Raney (0,1 éq.) 50% dans l'eau. On agite le milieu sous environ 5 atmosphères d'hydrogène à 60°C pendant 6 h, puis on le filtre sur terre de diatomées et on élimine le solvant par évaporation sous pression réduite. On purifie le résidu obtenu par chromatographie sur colonne de gel de silice en éluant par un mélange de chloroforme, méthanol et ammoniaque dans les proportions 90/10/1 pour fournir 1,231 g du composé désiré sous forme d'une cire jaune.
RMN ¹H (CDCl₃ ; 200 MHz): δ (ppm): 8,13 (d, 1H) ; 7,55 (dd, 1H) ; 6,80 (d, 1H) ; 6,45 (br s; 1H) ; 4,09 (q, 2H) ; 3,96 (s, 3H) ; 3,80-3,37 (AB, 2H) ; 2,52-1,88 (m, 8H) ; 1,21 (t, 3H).

### 1.3 3-[3-(6-méthoxypyridin-3-yl)pipéridin-3-yl]propan-1-ol

Dans un ballon de 250 ml, on introduit 2,321 g (7,58 mmoles) de 3-[3-(6-méthoxypyridin-3-yl)-6-oxopipéridin-3-yl]propanoate d'éthyle, obtenu à l'étape 1.2, en solution dans 128 ml de tétrahydrofurane anhydre à température ambiante. On additionne 2,876 g (75,8 mmoles) par portions d'hydrure d'aluminium et de lithium, le mélange résultant est ensuite agité à température ambiante pendant 3 heures. Ce dernier est ensuite hydrolysé par une solution aqueuse saturée en sulfate de sodium, filtré sur célite et concentré sous pression réduite. On purifie le résidu obtenu par chromatographie sur colonne de gel de silice en éluant par un mélange de chloroforme, méthanol et ammoniaque dans les proportions 90/10/1 pour fournir 1,80 g du composé désiré sous forme d'une cire jaune.
RMN ¹H (CDCl₃ ; 200 MHz) : δ (ppm): 7,94 (d, 1H) ; 7,37 (dd, 1H) ; 6,57 (d, 1H) ; 3,78 (s, 3H) ; 3,31 (t, 2H) ; 3,12-2,70 (AB, 2H) ; 2,65 (t, 2H) ; 2,11 (br s, 2H) ; 2,01-0,97 (m, 8H).

### 1.4 Bromhydrate (1 :1) de 5-[3-(3-bromopropyl)pipéridin-3-yl]pyridin-2-ol

Dans un tube scellé, on introduit 0,100 g (0,4 mmole) de 3-[3-(6-méthoxypyridin-3-yl)pipéridin-3-yl]propan-1-ol, obtenu à l'étape 1.3, en solution dans 5 ml d'acide bromhydrique aqueux (solution 48% massique). Le milieu réactionnel est agité à 110°C pendant 12 heures. Puis, cette solution est ramenée à température ambiante, concentrée sous pression réduite. Le résidu résultant est dissout dans le méthanol et évaporé sous pression réduite deux fois, trituré dans l'éther diéthylique pour conduire après filtration à 0,150 g du composé désiré sous forme d'une poudre marron.
Point de fusion : 230°C.
RMN ¹H (D₂O ; 200 MHz) : δ (ppm): 7,88 (dd, 1H) ; 7,51 (d, 1H) ; 6,80 (d, 1H) ; 3,65-3,27 (AB, 2H) ; 3,31 (t, 2H) ; 3,24-2,90 (m, 2H) ; 2,32-1,25 (m, 8H).

### 1.5 5-(1-azabicyclo[3.3.1]non-5-yl)pyridin-2-ol (Composé N°4)

Dans un ballon de 250 ml, on introduit 2,17 g (5,71 mmoles) de bromhydrate (1 :1) de 5-[3-(3-bromopropyl)pipéridin-3-yl]pyridin-2-ol, obtenu à l'étape 1.4, en solution dans 114 ml de chloroforme et 40 ml d'eau. On additionne 3,94 g (28,54 mmoles) de carbonate de sodium et le mélange réactionnel est agité à 80°C pendant 2 heures. Ce dernier est ensuite ramené à température ambiante, versé dans une ampoule à décanter et extrait deux fois par le chloroforme. Les phases organiques sont réunies, séchées sur sulfate de sodium, filtrées et concentrées sous pression réduite. Le résidu ainsi obtenu est trituré dans l'éther diéthylique pour conduire après filtration et séchage à 0,813 g du composé désiré sous la forme d'une poudre blanche.
Point de fusion : 191-192°C.
RMN ¹H (d⁶-DMSO ; 200 MHz) : δ (ppm): 7,89 (dd, 1H) ; 7,41 (d, 1H) ; 6,65 (d, 1H) ; 3,70-3,34 (m, 6H) ; 2,70-1,83 (m, 8H).

### Exemple 2 (Composé N°5)

### 5-(6-chloropyridin-3-yl)-1-azabicyclo[3.3.1]nonane

Dans un tube scellé, on introduit 1 g (4,59 mmoles) de 5-(1-azabicyclo[3.3.1]non-5-yl)pyridin-2-ol, obtenu à l'exemple 1, en solution dans 12,8 ml (137,7 mmoles) d'oxychlorure de phosphore et le tout est ensuite agité à 140°C pendant 45 minutes. Le mélange réactionnel est ensuite ramené à température ambiante, versé sur 200 g de glace pilée et agité 15 minutes. Puis, le milieu est ajusté très lentement à pH 10 par une solution de soude concentrée. Le tout est ensuite extrait deux fois par le chloroforme, les phases organiques sont ensuite réunies, séchées sur sulfate de sodium, filtrées et concentrées sous pression réduite pour fournir 461 mg du composé désiré sous forme d'une poudre marron.
Point de fusion : 245-246°C.
RMN ¹H (CDCl₃ ; 200 MHz) : δ (ppm): 8,29 (d, 1H) ; 7,55 (dd, 1H) ; 7,23 (d, 1H) ; 3,30-3,10 (m, 6H) ; 2,42-2,00 (m, 4H) ; 1,92-1,63 (m, 4H).

### Exemple 3 (Composé N°3)

### Chlorhydrate (2 : 1) de 5-[6-(3,4-diméthoxyphényl)pyridin-3-yl]-1-azabicyclo[3.3.1]nonane

Dans un ballon de 100 ml, on introduit 0,055 g (0,23 mmole) du 5-(6-chloropyridin-3-yl)-1-azabicyclo[3.3.1]nonane, obtenu à l'exemple 2, en solution dans 12 ml d'un mélange 1,2-diméthoxyéthane/eau 8/4. Puis, 0,106 g (0,58 mmole) d'acide 3,4-diméthoxyphényle boronique, 0,08 g (0,58 mmole) de carbonate de potassium et 0,024 g (0,03 mmole) de dichlorobis(triphénylphosphine)-palladium(II) sont introduits successivement sous atmosphère d'argon. On chauffe le mélange à 110°C pendant 2 heures, on le refroidit à température ambiante, et on le verse dans 300 ml d'une solution aqueuse saturée en carbonate de sodium. On extrait la phase aqueuse deux fois avec 100 ml de chloroforme, on sèche les phases organiques réunies sur sulfate de sodium et on les concentre sous pression réduite. On purifie le résidu par chromatographie sur colonne de gel de silice en éluant par un mélange chloroforme, méthanol et ammoniaque dans les proportions 90/10/1. Le composé obtenu sous forme de base (5-[6-(3,4-diméthoxyphényl)pyridin-3-yl]-1-azabicyclo[3.3.1]nonane) est transformé est chlorhydrate en ajoutant deux équivalents d'acide chlorhydrique en solution (5-6 N) dans l'alcool isopropylique. On obtient après trituration par l'éther diéthylique 0,035 g de produit attendu sous forme d'une poudre jaune.
Point de fusion : 235-236°C.
RMN ¹H (*d*⁶-DMSO) δ (ppm): 8,57 (s, 1H); 7,84-7,50 (m, 4H); 7,01 (d, 1H); 3,82 (s, 3H); 3,77 (s, 3H); 3,30 (br s, 2H); 3,13-2,83 (m, 4H); 2,31-1,95 (m, 4H); 1,88-1,61 (m, 2H); 1,55-1,33 (m, 2H).

### Exemple-4 (composé N°15)

### 5-(1-azabicyclo[2.2.1]hept-4-yl)pyridin-2-ol

### 4.1 3-cyano-3-(6-méthoxypyridin-3-yl)pentanedioate de diéthyle

Dans un ballon tricol de 100 ml on introduit 10,12 ml (20,25 mmoles) de diisopropylamidure de lithium dans 22 ml de tétrahydrofurane anhydre et sous atmosphère d'argon à -78°C. On additionne 1,00 g (6,75 mmoles) de (6-méthoxypyridin-3-yl)-acétonitrile (WO2004/111031) en solution dans 5 ml de tétrahydrofurane anhydre. Le mélange réactionnel est ensuite ramené doucement à 0°C et agité à cette température pendant une heure. Puis, le milieu réactionnel est refroidit à -78°C, et on ajoute 2,25 ml (20,25 mmoles) de bromure d'acétate d'éthyle au goutte à goutte. Le mélange résultant est ensuite lentement ramené à température ambiante puis laissé sous agitation 12 heures. Le milieu réactionnel est ensuite versé dans une solution aqueuse saturée en chlorure d'ammonium, extrait deux fois par de l'éther diéthylique. Les phases organiques sont réunies, séchées sur sulfate de sodium, filtrées et concentrées sous pression réduite. On purifie le résidu obtenu par chromatographie sur colonne de gel de silice en éluant par un mélange de cyclohexane et d'acétate d'éthyle dans les proportions 90/10.
On obtient 1,758 g de produit sous forme d'une huile marron.
RMN ¹H (CDCl₃ ; 200 MHz) : δ (ppm): 8,08 (d, 1H); 7,49 (dd, 1H); 6,55 (d, 1H); 3,90 (q, 4H); 3,72 (s, 3H); 3,11-2,79 (AB, 4H); 0.97 (t, 6H).

### 4.2 [3-(6-méthoxypyridin-3-yl)-5-oxopyrrolidin-3-yl]acétate d'éthyle

Dans un flacon d'hydrogénation on introduit 2,990 g (9,33 mmoles) de 3-cyano-3-(6-méthoxypyridin-3-yl)pentanedioate de diéthyle, tel que obtenu à l'étape 4.1, en solution dans 186 ml d'alcool éthylique, en présence de Nickel de Raney (0,1 éq.) 50% dans l'eau. On agite le milieu sous environ 5 atmosphères d'hydrogène à 60°C pendant 6 h, puis on le filtre sur terre de diatomées et on élimine le solvant par évaporation sous pression réduite. On purifie le résidu obtenu par chromatographie sur colonne de gel de silice en éluant par un mélange de chloroforme, méthanol et ammoniaque dans les proportions 90/10/1 pour fournir 1,5 g du composé désiré sous forme d'une cire jaune.
RMN ¹H (CDCl₃ ; 200 MHz) : δ (ppm): 7,93 (d, 1H); 7,37 (dd, 1H); 6,66 (d, 1H); 6,52 (br s, 1H); 3,91 (q, 2H); 3,85 (s, 3H); 3,84-3,57 (AB, 2H); 2,71 (t, 4H); 1,04 (t, 3H).

### 4.3 2-[3-(6-méthoxypyridin-3-yl)pyrrolidin-3-yl]éthanol

Dans un ballon de 250 ml, on introduit 1,50 g (5,39 mmoles) de [3-(6-méthoxypyridin-3-yl)-5-oxopyrrolidin-3-yl]acétate d'éthyle, obtenu à l'étape 4.2, en solution dans 90 ml de tétrahydrofurane anhydre à température ambiante. On additionne 2,046 g (53,90 mmoles) par portions d'hydrure d'aluminium et de lithium, le mélange résultant est ensuite agité à température ambiante pendant 3 heures. Ce dernier est ensuite hydrolysé par une solution aqueuse saturée en sulfate de sodium, filtré sur célite et concentré sous pression réduite. On purifie le résidu obtenu par chromatographie sur colonne de gel de silice en éluant par un mélange de chloroforme, méthanol et ammoniaque dans les proportions 90/10/1 pour fournir 0,41 g du composé désiré sous forme d'une cire jaune.
RMN ¹H (CDCl₃ ; 200 MHz) : δ (ppm): 7,95 (d, 1H) ; 7,39 (dd, 1H) ; 6,64 (d, 1H) ; 3,83 (s, 3H) ; 3,60-2,91 (m, 8H); 2,09 (t, 2H); 1,92-1,64 (m, 2H).

### 4.4 Bromhydrate (1 :1) de 5-[3-(2-bromoéthyl)pyrrolidin-3-yl]pyridin-2-ol

Dans un tube scellé, on introduit 0,32 g (1,44 mmoles) de 2-[3-(6-méthoxypyridin-3-yl)pyrrolidin-3-yl]éthanol, obtenu à l'étape 4.3, en solution dans 7,2 ml d'acide bromhydrique aqueux (solution 48% massique). Le milieu réactionnel est agité à 160°C pendant 5 heures. Puis, cette solution est ramenée à température ambiante, concentrée sous pression réduite. Le résidu résultant est dissout dans le méthanol et évaporé sous pression réduite deux fois, trituré dans l'éther diéthylique pour conduire après filtration à 0,5 g du composé désiré sous forme d'une gomme marron.
RMN ¹H (D₂O ; 200 MHz) : δ (ppm): 7,72 (dd, 1H) ; 7,49 (d, 1H) ; 6,65 (d, 1H) ; 3,78-2,90 (m, 6H) ; 2,55-2,05 (m, 4H).

### 4.5 5-(1-azabicyclo[2.2.1]hept-4-yl)pyridin-2-ol (Composé N°15)

Dans un ballon de 250 ml, on introduit 1,5 g (4,26 mmoles) de Bromhydrate (1 :1) de 5-[3-(2-bromoéthyl)pyrrolidin-3-yl]pyridin-2-ol, obtenu à l'étape 4.4, en solution dans 85 ml de chloroforme et 10 ml d'eau. On additionne 2,944 g (21,3 mmoles) de carbonate de sodium et le mélange réactionnel est agité à 60°C pendant 3 heures. Ce dernier est ensuite ramené à température ambiante, versé dans une ampoule à décanter et extrait deux fois par le chloroforme. Les phases organiques sont réunies, séchées sur sulfate de sodium, filtrées et concentrées sous pression réduite. Le résidu ainsi obtenu est trituré dans l'éther diéthylique pour conduire après filtration et séchage à 0,265 g du composé désiré sous la forme d'une poudre marron.
Point de fusion : 170-172°C.
RMN ¹H (d⁶-DMSO ; 200 MHz) : δ (ppm): 7,48 (dd, 1H) ; 7,08 (d, 1H) ; 6,27 (d, 1H) ; 2,94-2,74 (m, 2H) ; 2,65-2,42 (m, 2H) ; 2,35 (s, 2H) ; 1,75-1,50 (m, 2H) ; 1,50-1,33 (m, 2H).

### Exemple 5 (composé N°1)

### Chlorhydrate (2: 1) de 4-[6-(1-méthyl-1H-pyrazol-4-yl)pyridin-3-yl]-1-azabicyclo[2.2.1]heptane

### 5.1 4-(6-chloropyridin-3-yl)-1-azabicyclo[2.2.1]heptane

Dans un tube scellé, on introduit 0,37 g (1,94 mmoles) de 5-(1-azabicyclo[2.2.1]hept-4-yl)pyridin-2-ol, obtenu à l'exemple 4, en solution dans 5,44 ml (58,35 mmoles) d'oxychlorure de phosphore et le tout est ensuite agité à 140°C pendant 45 minutes. Le mélange réactionnel est ensuite ramené à température ambiante, versé sur 200 g de glace pilée et agité 15 minutes. Puis, le milieu est ajusté très lentement à pH 10 par une solution de soude concentrée. Le tout est ensuite extrait deux fois par le chloroforme, les phases organiques sont ensuite réunies, séchées sur sulfate de sodium, filtrées et concentrées sous pression réduite pour fournir 370 mg du composé désiré sous forme d'une poudre marron.
Point de fusion : 70-72°C.
RMN ¹H (DMSO ; 200 MHz) : δ (ppm): 8,36 (d, 1H); 7,80 (dd, 1H); 7,41 (d, 1H); 3,00-2,79 (m, 2H) ; 2,78-2,49 (m, 2H) ; 2,58 (s, 2H) ; 1,82-1,51 (m, 4H).

### 5.2 Chlorhydrate (2 : 1) de 4-[6-(1-méthyl-1H-pyrazol-4-yl)pyridin-3-yl]-1-azabicyclo[2.2.1]heptane (Composé N° 1).

Dans un ballon de 100 ml, on introduit 0,055 g (0,26 mmole) du 4-(6-chloropyridin-3-yl)-1-azabicyclo[2.2.1]heptane, obtenu à l'étape 5.1, en solution dans 12 ml d'un mélange 1,2-diméthoxyéthane/eau 8/4. Puis, 0,137 g (0,66 mmole) de 1-méthyl-4-(4,4,5,5-tetraméthyl-1,3,2dioxaborolan-2-yl)-1*H*-pyrazole, 0,091 g (0,66 mmole) de carbonate de potassium et 0,028 g (0,04 mmole) de dichlorobis(triphénylphosphine)-palladium(II) sont introduits successivement sous atmosphère d'argon. On chauffe le mélange à 110°C pendant 2 heures, on le refroidit à température ambiante, et on le verse dans 300 ml d'une solution aqueuse saturée en carbonate de sodium. On extrait la phase aqueuse deux fois avec 100 ml de chloroforme, on sèche les phases organiques réunies sur sulfate de sodium et on les concentre sous pression réduite. On purifie le résidu par chromatographie sur colonne de gel de silice en éluant par un mélange chloroforme, méthanol et ammoniaque dans les proportions 90/10/1. Le composé obtenu sous forme de base (4-[6-(1-méthyl-1*H*-pyrazol-4-yl)pyridin-3-yl]-1-azabicyclo[2.2.1]heptane) est transformé en chlorhydrate en ajoutant deux équivalents d'acide chlorhydrique en solutuion (5-6 N) dans l'alcool isopropylique. On obtient après trituration par l'éther diéthylique 0,053 g (62%) de produit attendu sous forme d'une poudre blanche.
Point de fusion : 281-283°C.
RMN ¹H (*d*⁶-DMSO) δ (ppm): 8,67 (s, 1H); 8,55 (d, 1H); 8,35 (s, 1H); 8,22 (d, 1H); 8,04 (d, 1H); 3,90 (s, 3H); 3,60 (s, 2H); 3,55-3,31 (m, 4H); 2,31-2,07 (m, 4H).

### Exemple 6 (Composé N° 13)

### 4-[6-(4-benzylpipérazin-1-yl)pyridin-3-yl]-1-azabicyclo[2.2.1]heptane

Dans un ballon de 50 ml sous argon contenant 0,036 g (0,37 mmole) de *tert-*butanolate de sodium en solution dans 5 ml de toluène préalablement bullés à l'argon, on introduit 0,055 g (0,32 mmole) de 1-benzyl-pipérazine. Puis, sont successivement additionnés sous atmosphère d'argon, 0,06 g (0,29 mmole) de 4-(6-chloropyridin-3-yl)-1-azabicyclo[2.2.1]heptane, obtenu à l'étape 5.1 de l'exemple 5, 0,003 g (0,01 mmole) de palladium(II) acétate et 0,011 g (0,03 mmole) de 2-dicyclohexylphosphino-2'-(N,N-diméthylamino)biphényl. On chauffe le mélange à 100°C pendant 3 heures, on le refroidit à température ambiante, dilué avec 40 ml de dichlorométhane,et on le verse dans 300 ml d'une solution aqueuse saturée en carbonate de sodium. On extrait la phase aqueuse deux fois avec 100 ml de dichlorométhane, on sèche les phases organiques réunies sur sulfate de sodium et on les concentre sous pression réduite. On purifie le résidu par chromatographie sur colonne de gel de silice en éluant par un mélange chloroforme, méthanol et ammoniaque dans les proportions 90/10/1. On obtient 0,04 g de produit attendu sous forme d'une poudre beige.
Point de fusion : 109-111°C.
RMN ¹H (*d*⁶-DMSO) δ (ppm): 8,05 (d, 1H) ; 7,49 (dd, 1H) ; 7,38-7,15 (m, 5H) ; 6,73 (d, 1H) ; 3,50 (s, 2H) ; 3,40 (br t, 4H) ; 3,01-2,79 (m, 2H) ; 2,69-2,35 (m, 8H) ; 1,78-1,42 (m, 4H).

### Exemple 7 (Composé N° 29)

### 4-(6-pyrrolidin-1-ylpyridin-3-yl)-1-azabicyclo[2.2.1]heptane

Dans un tube scellé contenant 0,060 g (0,29 mmole) de 4-(6-chloropyridin-3-yl)-1-azabicyclo[2.2.1]heptane, obtenu à l'étape 5.1 de l'exemple 5, est additionné 0,852 g (11,98 mmoles) de pyrrolidine. On chauffe le mélange à 130°C pendant 3 heures, on le refroidit à température ambiante, dilué avec 40 ml de toluène et le tout est concentré sous pression réduite. Le résidu ainsi obtenu est dilué avec 50 ml de dichlorométhane et on le verse dans 300 ml d'une solution aqueuse saturée en carbonate de sodium. On extrait la phase aqueuse deux fois avec 100 ml de dichlorométhane, on sèche les phases organiques réunies sur sulfate de sodium et on les concentre sous pression réduite. On purifie le résidu par chromatographie sur colonne de gel de silice en éluant par un mélange chloroforme, méthanol et ammoniaque dans les proportions 85/15/1,5. On obtient 0,029 g de produit attendu sous forme d'une poudre brune.
Point de fusion : 119-121°C.
RMN ¹H (*d*⁶-DMSO) δ (ppm): 7,99 (d, 1H) ; 7,42 (dd, 1H) ; 6,35 (d, 1H) ; 3,43-3,18 (m, 6H) ; 2,96-2,78 (m, 2H) ; 2,62-2,44 (m, 2H) ; 2,00-1,82 (m, 4H) ; 1,75-1,41 (m, 4H).

### Exemple 8 (Composé N° 62)

### 4-[6-(thiazol-2-yl)pyridin-3-yl]-1-azabicyclo[2.2.1]heptane

Dans un ballon de 50 ml sous argon contenant 0,055g (0,26 mmole) de 4-(6-chloropyridin-3-yl)-1-azabicyclo[2.2.1]heptane, obtenu à l'étape 5.1 de l'exemple 5, en solution dans 10 ml de tétrahydrofurane, sont successivement introduits 0,246 g (0,66 mmole) de 2-tributylstannylthiazole et 0,055 g (0,08 mmole) de bis(triphénylphosphine)palladium(II) chloride. On chauffe le mélange à reflux pendant 12 heures, on le refroidit à température ambiante, dilué avec 40 ml de dichlorométhane et on le verse dans 300 ml d'une solution aqueuse saturée en chlorure d'ammonium. La phase aqueuse est ajustée à pH = 5 avec une solution d'acide chlorhydrique 1 N. La phase organique est séparée, puis la phase aqueuse est ajustée à pH = 10 avec du carbonate de sodium en poudre. On extrait la phase aqueuse deux fois avec 100 ml de dichlorométhane, on sèche les phases organiques réunies sur sulfate de sodium et on les concentre sous pression réduite. On purifie le résidu par chromatographie sur colonne de gel de silice en éluant par un mélange chloroforme, méthanol et ammoniaque dans les proportions 95/5/0.5. On obtient 0,038 g de produit attendu sous forme d'une poudre blanche.
Point de fusion : 127-129°C.
RMN ¹H (*d*⁶-DMSO) δ (ppm): 8,60 (d, 1H); 8,04 (d, 1H); 7,98-7,82 (m, 2H); 7,77 (d, 1H); 3,02-2,76 (m, 2H); 2,70-2,33 (m, 4H); 1,90-1,51 (m, 4H).

Le tableau 1 qui suit illustre les structures chimiques et les propriétés physiques de quelques exemples de composés selon l'invention. Dans la colonne "Sel" de ce tableau, "-" désigne un composé à l'état de base, "HBr" désigne un bromhydrate et "HCl" désigne un chlorhydrate. Les rapports molaires acide:base sont indiqués en regard. « BOC » désigne un groupement *t*-butyloxycarbonyle.

**Tableau 1**

| | | | | | | |
|---|---|---|---|---|---|---|
| 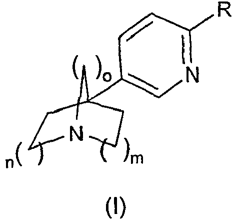 | | | | | | |

| **N°** | **n** | **m** | **o** | **R** | **Sel** | **PF (°C)** (Point de fusion) |
|---|---|---|---|---|---|---|
| **1** | 1 | 1 | 1 | 1-méthyl-1H-pyrazol-4-yl | HCl 2:1 | 281-283 |
| **2** | 2 | 2 | 1 | 1-méthyl-1H-pyrazol-4-yl | HCl 2:1 | 271-273 |
| **3** | 2 | 2 | 1 | 3,4-diméthoxyphényl | HCl 2:1 | 235-236 |
| **4** | 2 | 2 | 1 | OH | - | 191-192 |
| **5** | 2 | 2 | 1 | Cl | - | 245-246 |
| **6** | 1 | 1 | 1 | 1-méthyl-1H-indol-5-yl | - | 186-187 |
| **7** | 2 | 2 | 1 | pyridin-4-yl | - | 134-136 |
| **8** | 1 | 1 | 1 | 4-fluorophényl | - | 147-149 |
| **9** | 1 | 1 | 1 | 1-benzofur-2-yl | - | 164-165 |
| **10** | 2 | 2 | 1 | 1-benzofur-2-yl | - | 152-154 |
| **11** | 1 | 1 | 1 | pyridin-4-yl | - | 254-256 |
| **12** | 1 | 1 | 1 | quinolin-6-yl | HCl 2:1 | 327-328 |
| **13** | 1 | 1 | 1 | 4-benzylpipérazin-1-yl | - | 109-111 |
| **14** | 1 | 1 | 1 | 1-benzothièn-2-yl | - | 202-203 |
| **15** | 1 | 1 | 1 | OH | - | 170-172 |
| **16** | 1 | 1 | 1 | 1,3-benzodioxol-5-yl | - | 256-258 |
| **17** | 1 | 1 | 1 | 3,4-diméthoxyphényl | HCl 2:1 | 247-249 |
| **18** | 1 | 1 | 1 | 3-(trifluorométhyl)phényl | HCl 2:1 | 191-192 |
| **19** | 1 | 1 | 1 | 3-fluorophényl | - | 88-90 |
| **20** | 2 | 2 | 1 | 3-fluorophényl | - | 102-104 |
| **21** | 1 | 1 | 1 | 3-furyl | - | 279-281 |
| **22** | 1 | 1 | 1 | Dibenzo[b,d]fur-4-yl | - | 158-160 |
| **23** | 1 | 1 | 1 | quinolin-3-yl | - | 267-268 |
| **24** | 1 | 1 | 1 | pyridin-3-yl | - | 228-229 |
| **25** | 1 | 1 | 1 | 4-(trifluorométhoxy)phényl | - | 75-76 |
| **26** | 2 | 2 | 1 | 1-benzothièn-2-yl | - | 176-178 |
| **27** | 1 | 1 | 1 | 4-fluoro-2-méthoxyphényl | - | 169-170 |
| **28** | 1 | 1 | 1 | 1H-pyrrol-3-yl | HCl 2 : 1 | 324-326 |
| **29** | 1 | 1 | 1 | pyrrolidin-1-yl | - | 119-121 |
| **30** | 2 | 2 | 1 | dibenzo[b,d]fur-4-yl | - | 142-144 |
| **31** | 2 | 2 | 1 | 1,3-benzodioxol-5-yl | - | 124-126 |
| **32** | 2 | 2 | 1 | quinolin-6-yl | - | 151-153 |
| **33** | 2 | 2 | 1 | 1-méthyl-1H-indol-5-yl | - | 166-168 |
| **34** | 2 | 2 | 1 | 1H-pyrrol-3-yl | - | 152-154 |
| **35** | 2 | 2 | 1 | 3-(trifluorométhyl)phényl | HBr 2 : 1 | 155-157 |
| **36** | 2 | 2 | 1 | 4-fluorophényl | - | 107-109 |
| **37** | 1 | 1 | 1 | pyrimidin-5-yl | - | 244-246 |
| **38** | 1 | 1 | 1 | 2,4-diméthoxypyrimidin-5-yl | - | 104-106 |
| **39** | 2 | 2 | 1 | biphényl-4-yl | - | 146-148 |
| **40** | 2 | 2 | 1 | 3-furyl | - | 335-337 |
| **41** | 2 | 2 | 1 | pyridin-3-yl | - | 118-120 |
| **42** | 2 | 2 | 1 | 4-(trifluorométhoxy)phényl | - | 120-122 |
| **43** | 2 | 2 | 1 | 4-fluoro-2-méthoxyphényl | - | 148-150 |
| **44** | 2 | 2 | 1 | quinolin-3-yl | - | 160-162 |
| **45** | 1 | 1 | 1 | biphényl-4-yl | - | 191-192 |
| **46** | 1 | 1 | 1 | quinolin-8-yl | - | 176-178 |
| **47** | 2 | 2 | 1 | 1H-1,2,3-benzotriazol-1-yl | HCl 1 : 1 | 258-260 |
| **48** | 2 | 2 | 1 | Dibenzo[b,d]thièn-2-yl | HCl 2 : 1 | 242-244 |
| **49** | 2 | 2 | 1 | 4-méthoxyphényl | - | 138-139 |
| **50** | 2 | 2 | 1 | 3-méthoxyphényl | HCl 2 : 1 | 267-269 |
| **51** | 1 | 1 | 1 | 2,1,3-benzoxadiazol-5-yl | - | 139-140 |
| **52** | 1 | 1 | 1 | 2-morpholin-4-ylpyrimidin-5-yl | - | 226-227 |
| **53** | 1 | 1 | 1 | 1-méthyl-1*H*-pyrrol-2-yl | HCl 2 : 1 | 248-250 |
| **54** | 1 | 1 | 1 | 3-fluoro-5-méthoxyphényl | - | 95-97 |
| **55** | 1 | 1 | 1 | 3-fluoro-4-méthoxyphényl | - | 125-126 |
| **56** | 1 | 1 | 1 | 2-fluoro-3-méthoxyphényl | - | 144-146 |
| **57** | 2 | 2 | 1 | 3-fluoro-4-méthoxyphényl | - | 138-140 |
| **58** | 2 | 2 | 1 | 2-fluoro-3-méthoxyphényl | - | 144-146 |
| **59** | 2 | 2 | 1 | 1-méthyl-1H-pyrrol-2-yl | - | 105-107 |
| **60** | 2 | 2 | 1 | 2-furyl | HCl 2: 1 | 292-294 |
| **61** | 2 | 2 | 1 | 3-(1H-pyrazol-1-yl)phényl | - | 135-137 |
| **62** | 1 | 1 | 1 | thiazol-2-yl | - | 127-129 |
| **63** | 2 | 2 | 1 | pyrimidin-5-yl | - | 139-141 |
| **64** | 2 | 2 | 1 | 1-naphthyl | - | 167-169 |
| **65** | 2 | 2 | 1 | 2-morpholin-4-ylpyrimidin-5-yl | - | 189-191 |
| **66** | 2 | 2 | 1 | 2,4-diméthoxypyrimidin-5-yl | - | 81-83 |
| **67** | 2 | 2 | 1 | 2,1,3-benzoxadiazol-5-yl | - | 151-153 |
| **68** | 2 | 2 | 1 | phényl | - | 103-105 |
| **69** | 2 | 2 | 1 | 2,5-diméthoxyphényl | - | 101-103 |
| **70** | 2 | 2 | 1 | 4-(trifluorométhyl)phényl | - | 135-137 |
| **71** | 2 | 2 | 1 | 6-méthoxypyridin-3-yl | - | 134-136 |
| **72** | 2 | 2 | 1 | isoquinolin-4-yl | HBr 2 : 1 | 332-334 |
| **73** | 2 | 2 | 1 | 3,5-bis(trifluorométhyl)phényl | HBr 2 : 1 | 226-228 |
| **74** | 2 | 2 | 1 | thiazol-2-yl | - | 124-126 |
| **75** | 2 | 2 | 1 | 4-phénoxyphényl | - | 129-131 |
| **76** | 2 | 2 | 1 | 2-fluorophényl | - | 117-119 |
| **77** | 2 | 2 | 1 | (4-N-pyrrolidine)pyridin-3-yl | - | 301-303 |
| **78** | 2 | 2 | 1 | (N-(N'-BOC)pipérazine)-pyridin-3-yl | - | 202-204 |
| **79** | 2 | 2 | 1 | 2-méthoxyphényl | HBr 2 : 1 | 239-241 |
| **80** | 2 | 2 | 1 | 2,4-diméthoxyphényl | HBr 2 : 1 | 198-200 |
| **81** | 2 | 2 | 1 | 3-(trifluorométhoxy)phényl | HBr 2 : 1 | 267-269 |
| **82** | 2 | 2 | 1 | 3-(N,N-diméthylamino)phényl | HBr 3 : 1 | 256-258 |
| **83** | 1 | 1 | 1 | isoquinolin-4-yl | - | 143-145 |
| **84** | 1 | 1 | 1 | isoquinolin-5-yl | - | 176-178 |
| **85** | 1 | 1 | 1 | phenyl | - | 126-128 |
| **86** | 1 | 1 | 1 | 3,5-bis(trifluorométhyl)phényl | - | 117-119 |
| **87** | 1 | 1 | 1 | 6-méthoxypyridin-3-yl | - | 97-99 |
| **88** | 1 | 1 | 1 | (N-(N'-BOC)pipérazine)-pyridin-3-yl | - | 216-218 |
| **89** | 2 | 2 | 1 | isoquinolin-5-yl | HBr 3 : 1 | 246-248 |
| **90** | 1 | 1 | 1 | 4-fluoropyridin-3-yl | - | 108-109 |
| **91** | 2 | 2 | 1 | 2-fluoropyridin-3-yl | - | 98-99 |
| **92** | 1 | 1 | 1 | 2-méthoxyphényl | HBr 2 : 1 | 114-116 |
| **93** | 1 | 1 | 1 | 2,4-diméthoxyphényl | HBr 2 : 1 | 195-197 |
| **94** | 1 | 1 | 1 | 2,5-diméthoxyphényl | HBr 2 : 1 | 152-154 |
| **95** | 1 | 1 | 1 | 2-fluorophényl | - | 110-112 |
| **96** | 1 | 1 | 1 | 4-méthoxyphényl | - | 160-162 |
| **97** | 1 | 1 | 1 | 2-naphthyl | - | 154-156 |
| **98** | 1 | 1 | 1 | 4-phénoxyphényl | - | 141-143 |
| **99** | 1 | 1 | 1 | 2-(trifluorométhyl)phényl | HBr 2: 1 | 310-312 |
| **100** | 2 | 2 | 1 | 2-naphthyl | - | 163-165 |
| **101** | 1 | 1 | 1 | 2-fluoropyridin-3-yl | - | 116-117 |
| **102** | 2 | 2 | 1 | 4-fluoropyridin-3-yl | - | 121-122 |
| **103** | 1 | 1 | 1 | 3-trifluorométhoxy-phényl | HCl 1 : 1 | 152-154 |
| **104** | 2 | 2 | 1 | quinolin-5-yl | - | 147-149 |
| **105** | 2 | 2 | 1 | 2-fluoro-biphényl-4-yl | - | 144 |
| **106** | 2 | 2 | 1 | 3-acétyl-phényl | HBr 2 : 1 | 284-285 |
| **107** | 1 | 1 | 1 | 3-acétyl-phényl | - | 95-98 |
| **108** | 1 | 1 | 1 | 4-méthyl-phényl | - | 134-136 |
| **109** | 1 | 1 | 1 | 2-fluoro-biphényl-4-yl | - | 153-155 |
| **110** | 1 | 1 | 1 | quinolin-5-yl | - | 129-131 |
| **111** | 1 | 1 | 1 | 3-méthoxy-phényl | - | 87-89 |
| **112** | 1 | 1 | 1 | 3,4-difluoro-phényl | - | 85-87 |
| **113** | 1 | 1 | 1 | 3-pyrazol-1-yl-phényl | - | 114-116 |

Les composés de l'invention ont fait l'objet d'essais pharmacologiques qui ont mis en évidence leur intérêt comme substances actives de médicaments.

Ainsi ils ont été étudiés quant à leur affinité vis-à-vis des récepteurs nicotiniques contenant la sous unité α₇, selon les méthodes décrites par Mark et Collins dans *J. Pharmacol. Exp. Ther.* 1982, **22,** 564 et par Marks et coll. dans Mol. *Pharmacol.* 1986,30,427.
On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 G pendant 10 min. On élimine le culot et on centrifuge le surnageant à 8000 G pendant 20 min à 4 °C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.
Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]-α-bungarotoxine à 1 nM dans un volume final de 250 µl de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale ; la liaison non spécifique représente environ 60 % de la liaison totale récupérée sur le filtre. Pour chaque concentration de composé étudié on détermine le pourcentage d'inhibition de la liaison spécifique de [³H]-α-bungarotoxine, puis on calcule la Cl₅₀, concentration de composé qui inhibe 50% de la liaison spécifique. Les Cl₅₀ des composés de l'invention les plus affins se situent entre 0,001 et 1 µM. Les données expérimentales de quelques composés spécifiques sont indiquées dans le tableau 2 qui suit.

**Tableau 2**

| Composé N° | Cl₅₀ α₇ (nM) |
|---|---|
| 113 | 4,7 |
| 16 | 9,1 |
| 12 | 37,1 |
| 19 | 13,8 |

Les composés de l'invention ont aussi été étudiés quant à leur affinité vis à vis des récepteurs nicotiniques contenant la sous-unité α₄β₂ selon les méthodes décrites par Anderson et Arneric dans *Eur. J. Pharmacol.* 1994, **253,** 261 et par Hall et coll. dans *Brain Res.* 1993, **600,** 127.
On décapite des rats mâles Sprague Dawley de 150 à 200 g et on prélève rapidement la totalité du cerveau, on l'homogénéise dans 15 volumes d'une solution de sucrose à 0,32 M à 4°C, puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron ™ dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C.
Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM la liaison non spécifique représente 75 à 85% de la liaison totale récupérée sur le filtre. Pour chaque concentration de composé étudié on détermine le pourcentage d'inhibition de la liaison spécifique de [³H]-cytisine, à des doses de 1 µM et 10 µM. Pour les composés les plus affins de l'invention, on calcule la Cl₅₀, concentration de composé qui inhibe 50% de la liaison spécifique.
Les Cl₅₀ des composés de l'invention les plus affins se situent entre 0,001 et 1 µM.

Les composés de l'invention ont également été étudiés quant à leur affinité vis-à-vis des récepteurs nicotiniques périphériques de type ganglionnaire selon la méthode décrite par Houghtling et coll. dans Mol. Pharmacol. 1995, 48, 280.
On décongèle des glandes surrénales de boeuf conservées à -80°C, et on les homogénéise à l'aide d'un broyeur Polytron ™ dans 20 volumes de tampon Tris-HCl 50 mM à pH 7,4 et à 4°C, puis on les centrifuge à 35000 G pendant 10 min. On élimine le surnageant et on remet le culot en suspension dans 30 volumes de tampon Tris-HCl 50 mM à 4°C et on ré-homogénéise avant de re-centrifuger à 35000 G pendant 10 min. On reprend le dernier culot dans 10 volumes de tampon Tris-HCl à 4°C. On fait incuber 100 µl de membrane soit 10 mg de tissu frais à 24°C pendant 3h en présence de 50 µl de [³H]-epibatidine 0,66 nM finale dans un volume final de 250 µl de tampon, en présence ou en absence de composé à tester. On arrête la réaction par dilution des échantillons avec du tampon Tris-HCl 50 µM pH 7,4 à 4°C puis on filtre sur des filtres Whatman GF/C™ préalablement traités pendant 3 heures avec de la polyéthylènimine à 0,5%. On rince les filtres deux fois avec 5 ml de tampon et on mesure la radioactivité retenue sur le filtre par scintigraphie liquide. On détermine la liaison non spécifique en présence de (-)-nicotine 2 mM finale; la liaison non spécifique représente 30 à 40% de la liaison totale récupérée sur le filtre. Pour chaque concentration de produit étudié on détermine le pourcentage d'inhibition de la liaison spécifique de [³H]-epibatidine, puis on calcule la Cl₅₀, concentration de composé qui inhibe 50% de la liaison spécifique.
Les Cl₅₀ des composés de l'invention se situent entre 0,001 et 1 µM.

Les résultats obtenus montrent que certains composés de l'invention sont des ligands sélectifs pour la sous-unité α₇ du récepteur nicotinique et que d'autres sont mixtes α₄β₂ et α₇.

Ces résultats suggèrent l'utilisation des composés dans le traitement ou la prévention des désordres liés à un dysfonctionnement des récepteurs nicotiniques, notamment au niveau du système nerveux central mais aussi au niveau du système périphérique.

Ces désordres comprennent les altérations cognitives, plus spécifiquement mnésiques (acquisition, consolidation et rappel), mais également les atteintes des processus attentionnels, et les troubles des fonctions exécutives liées à la maladie d'Alzheimer, au vieillissement pathologique (Age Associated Memory Impairment, AAMI) ou normal (senile dementia), au syndrome Parkinsonien, à la trisomie 21 (Down's syndrome), aux pathologies psychiatriques, en particulier les troubles cognitifs associés à la schizophrénie (Cognitive Impairment Associated with Schizophrenia, CIAS), les troubles liés au stress post-traumatique (Post-Traumatic Stress Disorder, PTSD), au syndrome alcoolique de Korsakoff, aux démences vasculaires (multi-infarct dementia, MDI), aux traumatismes crâniens.

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Ils pourraient aussi présenter une activité thérapeutique neuro-protectrice vis à vis des atteintes anatomo-histo-pathologiques liées aux maladies neuro-dégénératives susnommées.

Ils pourraient également être utiles dans le traitement de la sclérose en plaques.

Les composés de l'invention pourraient également constituer un traitement curatif ou symptomatique des accidents vasculaires cérébraux et des épisodes hypoxiques cérébraux. Ils peuvent être utilisés dans les cas de pathologies psychiatriques : schizophrénie (symptômes positifs et/ou négatifs), troubles bipolaires, dépression, anxiété, attaques de panique, PTSD, troubles de l'attention avec hyperactivité (Attention Deficit Hyperactivity Disorder, ADHD), comportements compulsifs et obsessionnels.

Ils pourraient prévenir les symptômes dus au sevrage au tabac, à l'alcool, aux différentes substances induisant une dépendance, telles que cocaïne, LSD, cannabis, benzodiazépines.

Ils pourraient être utiles dans le traitement de la douleur d'origine diverse (y compris les douleurs chroniques, neuropathiques ou inflammatoires).

Par ailleurs les composés de l'invention pourraient être utilisés pour le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse, des chocs septiques.

Les composés de l'invention pourraient aussi être utilisés pour le traitement des processus inflammatoires d'origines diverses, en particulier les inflammations concernant le système nerveux central, l'inflammation pulmonaire liée aux allergies ou à l'asthme, la parodontite, la sarcoïdose, la pancréatite, les lésions de reperfusion, la polyarthrite rhumatoïde.

Les composés de l'invention pourraient également être utiles dans le traitement de pathologies dermatologiques telle que le psoriasis et dans le traitement de l'asthme.

Les composés de l'invention pourraient être aussi utilisés pour le traitement de la recto-colite ulcéro-hémorragique.

Les composés selon l'invention peuvent donc être utilisés pour la préparation de médicaments, en particulier de médicaments utiles dans le traitement ou la prévention des désordres liés à un dysfonctionnement des récepteurs nicotiniques, notamment des désordres ci-dessus mentionnés.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement ou la prévention des désordres liés à un dysfonctionnement des récepteurs nicotiniques, notamment des désordres ci-dessus mentionnés.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) 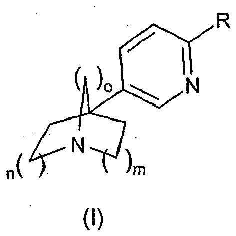 dans laquelle :
R représente
soit un atome d'hydrogène ou d'halogène ;
soit un groupe hydroxyle, (C₁-C₆)alcoxy, (C₃-C₇)cycloalkyle-O- ou (C₃-C₇)cycloalkyle-(C₁-C₃)alkylène-O- ;
soit un groupe hétérocycloalkyle, aryle ou hétéroaryle; ce groupe pouvant éventuellement être substitué par un ou plusieurs groupes choisis parmi les atomes d'halogènes, les groupes (C₁-C₆)alkyle, (C₃-C₇)cycloalkyle, (C₃-C₇)cycloalkyle-(C₁-C₃)alkylène, (C₁-C₆)alcoxy, (C₃-C₇)cycloalkyle-O-, (C₃-C₇)cycloalkyle-(C₁-C₃)alkylène-O-, (C₁-C₆)fluoroalkyle, (C₁-C₆)fluoroalcoxy, nitro, cyano, hydroxyle, amino, (C₁-C₆)alkylamino ou di(C₁-C₆)alkylamino, hétérocycloalkyle, aryle, aryle-(C₁-C₆)alkylène, hétéroaryle, hétéroaryle-(C₁-C₆)alkylène, aryle-O-, -C(O)-(C₁-C₆)alkyle,
le groupe hétérocycloalkyle étant éventuellement substitué par-C(O)O(CH₃)₃ ;
n représente 1 ou 2 ;
m représente 1 ou 2 ;
o représente 1 ou 2 ;
à l'état de base ou de sel d'addition à un acide, ainsi qu'à l'état d'hydrate ou de solvat ;
le cas où n et o =1 et m = 2 étant exclu ;
les composés suivants étant exclus:
- 4-(6-fluoropyridin-3-yl)-1-azabicyclo[2.2.1]heptane ;
- 4-(6-chloropyridin-3-yl)-1-azabicyclo[2.2.1]heptane ;
- 4-(6-bromopyridin-3-yl)-1-azabiçyclo[2.2.1]heptane ;
- 4-(pyridin-3-yl)-1-azabicyclo[2.2.1]heptane.

2. Composé de formule (I) selon la revendication 1, **caractérisé en ce que**
R représente
soit un atome d'hydrogène ou d'halogène ;
soit un groupe hydroxyle ;
soit un groupe hétérocycloalkyle, aryle ou hétéroaryle ; ce groupe pouvant éventuellement être substitué par un ou plusieurs groupes choisis parmi les atomes d'halogènes, les groupes (C₁-C₆)alkyle, (C₁-C₆)alcoxy, (C₁-C₆)fluoroalkyle, (C₁-C₆)fluoroalcoxy, nitro, cyano, hydroxyle, amino, (C₁-C₆)alkylamino ou di(C₁-C₆)alkylamino, hétérocycloalkyle, aryle, aryle-(C₁-C₆)alkylène, hétéroaryle, aryle-O-, -C(O)-(C₁-C₆)alkyle, le groupe hétérocycloalkyle étant éventuellement substitué par -C(O)O(CH₃)₃ ;
n représente 1 ou 2 ;
m représente 1 ou 2 ;
o représente 1 ou 2 ;
à l'état de base ou de sel d'addition à un acide, ainsi qu'à l'état d'hydrate ou de solvat.

3. Composé de formule (I) selon la revendication 1 ou 2, **caractérisé en ce que**
R représente
soit un atome d'halogène ;
soit un groupe hydroxyle ;
soit un groupe hétérocycloalkyle, soit un groupe aryle, soit un groupe hétéroaryle ; ce groupe pouvant éventuellement être substitué par un ou plusieurs groupes choisis parmi les atomes d'halogènes, les groupes (C₁-C₆)alkyle, (C₁-C₆)alcoxy, (C₁-C₆)fluoroalkyle, (C₁-C₆)fluoroalcoxy, di(C₁-C₆)alkylamino, hétérocycloalkyle, aryle, aryle-(C₁-C₆)alkylène, hétéroaryle, aryle-O-, -C(O)-(C₁-C₆)alkyle ; le groupe hétérocycloalkyle étant éventuellement substitué par -C(O)O(CH₃)₃ ;
n représente 1 ou 2 ;
m représente 1 ou 2 ;
o représente 1 ;
à l'état de base ou de sel d'addition à un acide, ainsi qu'à l'état d'hydrate ou de solvat.

4. Composé de formule (I) selon l'une quelconque des revendications 1 à 3, **caractérisé en ce que** n et m et o = 1 ; à l'état de base ou de sel d'addition à un acide, ainsi qu'à l'état d'hydrate ou de solvat.

5. Composé de formule (I) selon l'une quelconque des revendications 1 à 3, **caractérisé en ce que** n et m = 2 et o = 1 ; à l'état de base ou de sel d'addition à un acide, ainsi qu'à l'état d'hydrate ou de solvat.

6. Médicament, **caractérisé en ce qu'**il comprend un composé de formule (I) selon l'une quelconque des revendications 1 à 5, ou un sel d'addition de ce composé à un acide pharmaceutiquement acceptable, ou encore un hydrate ou un solvat.

7. Composition pharmaceutique, **caractérisée en ce qu'**elle comprend un composé de formule (I) selon l'une quelconque des revendications 1 à 5, ou un sel pharmaceutiquement acceptable, un hydrate ou un solvat de ce composé, ainsi qu'au moins un excipient pharmaceutiquement acceptable.

8. Utilisation d'un composé de formule (I) selon l'une quelconque des revendications 1 à 5 pour la préparation d'un médicament destiné au traitement et à la prévention des altérations cognitives ; des atteintes des processus attentionnels ; des troubles des fonctions exécutives liées à la maladie d'Alzheimer, au vieillissement pathologique ou normal, au syndrome Parkinsonien, à la trisomie 21, aux pathologies psychiatriques, au syndrome alcoolique de Korsakoff, aux démences vasculaires, aux traumatismes crâniens ; des troubles moteurs observés dans la maladie de Parkinson ou d'autres maladies neurologiques ou des atteintes anatomo-histo-pathologiques liées aux maladies neuro-dégénératives susnommées ; de la sclérose en plaques.

9. Utilisation d'un composé de formule (I) selon l'une quelconque des revendications 1 à 5 pour la préparation d'un médicament destiné au traitement et à la prévention des accidents vasculaires cérébraux, des épisodes hypoxiques cérébraux, des pathologies psychiatriques.

10. Utilisation d'un composé de formule (I) selon l'une quelconque des revendications 1 à 5 pour la préparation d'un médicament destiné à la prévention des symptômes dus au sevrage au tabac, à l'alcool, aux différentes substances induisant une dépendance.

11. Utilisation d'un composé de formule (I) selon l'une quelconque des revendications 1 à 5 pour la préparation d'un médicament destiné au traitement de la douleur.

12. Utilisation d'un composé de formule (I) selon l'une quelconque des revendications 1 à 5 pour la préparation d'un médicament destiné au traitement de l'ischémie des membres inférieurs, de l'artérite oblitérante des membres inférieurs, de l'ischémie cardiaque, de l'infarctus du myocarde, de l'insuffisance cardiaque, du déficit de cicatrisation cutanée des patients diabétiques, des ulcères variqueux de l'insuffisance veineuse, des chocs septiques.

13. Utilisation d'un composé de formule (I) selon l'une quelconque des revendications 1 à 5 pour la préparation d'un médicament destiné au traitement des processus inflammatoires.

14. Utilisation d'un composé de formule (I) selon l'une quelconque des revendications 1 à 5 pour la préparation d'un médicament destiné au traitement des pathologies dermatologiques, de l'asthme, de la recto-colite ulcéro-hémorragique.

## Claims

1. Compound corresponding to the formula (I) 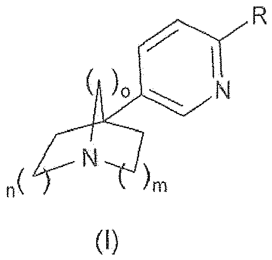 in which:
R represents
either a hydrogen or halogen atom;
or a hydroxyl, (C₁-C₆) alkoxy, (C₃-C₇)cycloalkyl-O- or (C₃-C₇)cycloalkyl-(C₁-C₃)alkylene-O- group;
or a heterocycloalkyl, aryl or heteroaryl group; it being possible for this group optionally to be substituted by one or more groups chosen from halogen atoms or (C₁-C₆)alkyl, (C₃-C₇)cycloalkyl, (C₃-C₇) cycloalkyl- (C₁-C₃)alkylene, (C₁-C₆)alkoxy, (C₃-C₇)cycloalkyl-O-, (C₃-C₇) cycloalkyl- (C₁-C₃) alkylene-O-, (C₁-C₆) - fluoroalkyl, (C₁-C₆)fluoroalkoxy, nitro, cyano, hydroxyl, amino, (C₁-C₆) alkylamino or (C₁-C₆) alkylamino, heterocycloalkyl, aryl, aryl-(C₁-C₆)alkylene, heteroaryl, heteroaryl-(C₁-C₆)alkylene, aryl-O- or -C(O) (C₁-C₆)alkyl groups, the heterocycloalkyl group optionally being substituted by -C(O)O(CH₃)₃;
n represents 1 or 2;
m represents 1 or 2;
o represents 1 or 2;
in the base form or in the form of an addition salt with an acid, and also in the hydrate or solvate form;
the case where n and o = 1 and m = 2 being excluded;
the following compounds being excluded:
- 4-(6-fluoropyridin-3-yl)-1-azabicyclo[2.2.1]heptane;
- 4-(6-chloropyridin-3-yl)-1-azabicyclo[2.2.1]heptane;
- 4-(6-bromopyridin-3-yl)-1-azabicyclo[2.2.1]heptane;
- 4-(pyridin-3-yl)-1-azabicyclo[2.2.1]heptane.

2. Compound of formula (I) according to Claim 1, **characterized in that**
R represents
either a hydrogen or halogen atom;
or a hydroxyl group;
or a heterocycloalkyl, aryl or heteroaryl group; it being possible for this group optionally to be substituted by one or more groups chosen from halogen atoms or (C₁-C₆)alkyl, (C₁-C₆)alkoxy, (C₁-C₆)fluoroalkyl, (C₁-C₆)fluoroalkoxy, nitro, cyano, hydroxyl, amino, (C₁-C₆)alkylamino or di(C₁-C₆)alkylamino, heterocycloalkyl, aryl, aryl-(C₁-C₆)alkylene, heteroaryl, aryl-O- or -C(O)-(C₁-C₆) alkyl groups, the heterocycloalkyl group optionally being substituted by -C (O) O (CH₃) ₃;
n represents 1 or 2;
m represents 1 or 2;
o represents 1 or 2;
in the base form or in the form of an addition salt with an acid, and also in the hydrate or solvate form.

3. Compound of formula (I) according to Claim 1 or 2, **characterized in that**
R represents
either a halogen atom;
or a hydroxyl group;
or a heterocycloalkyl group or an aryl group or a heteroaryl group; it being possible for this group optionally to be substituted by one or more groups chosen from halogen atoms or (C₁-C₆) alkyl, (C₁-C₆) alkoxy, (C₁-C₆) fluoroalkyl, (C₁-C₆) fluoroalkoxy, di(C₁-C₆)alkylamino, heterocycloalkyl, aryl, aryl-(C₁-C₆)alkylene, heteroaryl, aryl-O- or -C(O)-(C₁-C₆)alkyl groups; the heterocycloalkyl group optionally being substituted by -C(O)O(CH₃)₃;
n represents 1 or 2;
m represents 1 or 2;
o represents 1;
in the base form or in the form of an addition salt with an acid, and also in the hydrate or solvate form.

4. Compound of formula (I) according to any one of Claims 1 to 3, **characterized in that** n and m and o = 1; in the base form or in the form of an addition salt with an acid, and also in the hydrate or solvate form.

5. Compound of formula (I) according to any one of Claims 1 to 3, **characterized in that** n and m = 2 and o = 1; in the base form or in the form of an addition salt with an acid, and also in the hydrate or solvate form.

6. Medicament, **characterized in that** it comprises a compound of formula (I) according to any one of Claims 1 to 5 or an addition salt of this compound with a pharmaceutically acceptable acid or also a hydrate or a solvate.

7. Pharmaceutical composition, **characterized in that** it comprises a compound of formula (I) according to any one of Claims 1 to 5 or a pharmaceutically acceptable salt, a hydrate or a solvate of this compound, and also at least one pharmaceutically acceptable excipient.

8. Use of a compound of formula (I) according to any one of Claims 1 to 5 in the preparation of a medicament intended for the treatment and prevention of detrimental cognitive changes; attacks on attentional processes; disorders of the executive functions related to Alzheimer's disease, to pathological or normal ageing, to Parkinsonian syndrome, to trisomy 21, to psychiatric pathologies, to Korsakoff's alcoholic syndrome, to vascular dementias or to cranial traumas; motor disorders observed in Parkinson's disease or other neurological diseases or anatomic histopathological attacks related to the abovementinoed neurodegenerative diseases; or multiple sclerosis.

9. Use of a compound of formula (I) according to any one of Claims 1 to 5 in the preparation of a medicament intended for the treatment and prevention of strokes, cerebral hypoxic episodes or psychiatric pathologies.

10. Use of a compound of formula (I) according to any one of Claims 1 to 5 in the preparation of a medicament intended for the prevention of symptoms due to weaning from tobacco, from alcohol and from various dependence-inducing substances.

11. Use of a compound of formula (I) according to any one of Claims 1 to 5 in the preparation of a medicament intended for the treatment of pain.

12. Use of a compound of formula (I) according to any one of Claims 1 to 5 in the preparation of a medicament intended for the treatment of lower limb ischemia, lower limb arterial occlusive disease, cardiac ischemia, myocardial infarction, cardiac insufficiency, skin healing deficiency in diabetic patients, varicose ulcers of venous insufficiency, or septic shock.

13. Use of a compound of formula (I) according to any one of Claims 1 to 5 in the preparation of a medicament intended for the treatment of inflammatory processes.

14. Use of a compound of formula (I) according to any one of Claims 1 to 5 in the preparation of a medicament intended for the treatment of dermatological pathologies, asthma or ulcerative colitis.

## Patentansprüche

1. Verbindung der Formel (I) 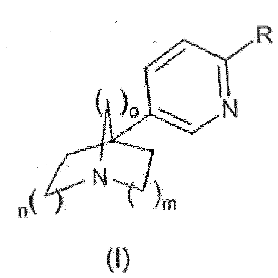 worin:
R für
ein Wasserstoff- oder Halogenatom;
oder eine Hydroxyl-, (C₁-C₆) -Alkoxy-, (C₃-C₇) - Cycloalkyl-O- oder (C₃-C₇) -Cycloalkyl- (C₁-C₃) - alkylen-O-Gruppe;
oder eine Heterocycloalkyl-, Aryl- oder Heteroarylgruppe; wobei diese Gruppe gegebenenfalls durch eine oder mehrere unter Halogenatomen und (C₁-C₆) -Alkyl-, (C₃-C₇) -Cycloalkyl-, (C₃-C₇) -Cycloalkyl- (C₁-C₃) -alkylen, (C₁-C₆) -Alkoxy-, (C₃-C₇) - Cycloalkyl-O-, (C₃-C₇)-Cycloalkyl(C₁-C₃)-alkylen-O-, (C₁-C₆) -fluoralkyl-, (C₁-C₆) -Fluoralkoxy-, Nitro-, Cyano-, Hydroxyl-, Amino-, (C₁-C₆)-Alkylamino- oder Di-(C₁-C₆)-alkylamino-, Heterocycloalkyl-, Aryl-, Aryl- (C₁-C₆) -alkylen-, Heteroaryl-, Heteroaryl-(C₁-C₆)-alkylen-, Aryl-O-oder -C(O)-(C₁-C₆)-Alkylgruppen ausgewählte Gruppen substituiert sein kann, wobei die Heterocycloalkylgruppe gegebenenfalls durch -C(O)O(CH₃)₃ substituiert ist;
steht;
n für 1 oder 2 steht;
m für 1 oder 2 steht;
o für 1 oder 2 steht;
in Basen- oder Säureadditionssalzform sowie in Hydrat- oder Solvatform;
wobei der Fall mit n und o = 1 und m = 2 ausgeschlossen ist;
wobei die folgenden Verbindungen ausgeschlossen sind:
- 4-(6-Fluorpyridin-3-yl)-1-azabicyclo[2.2.1]-heptan;
- 4-(6-Chlorpyridin-3-yl)-1-azabicyclo[2.2.1]-heptan;
- 4-(6-Brompyridin-3-yl)-1-azabicyclo[2.2.1]-heptan;
- 4-(Pyridin-3-yl)-1-azabicyclo[2.2.1]heptan.

2. Verbindung der Formel (I) nach Anspruch 1, **dadurch gekennzeichnet, dass**
R für
ein Wasserstoff- oder Halogenatom
oder eine Hydroxylgruppe
oder eine Heterocycloalkyl-, Aryl- oder Heteroarylgruppe; wobei diese Gruppe gegebenenfalls durch eine oder mehrere unter Halogenatomen und (C₁-C₆) -Alkyl-, (C₁-C₆) -Alkoxy-, (C₁-C₆) -Fluoralkyl-, (C₁-C₆) -Fluoralkoxy-, Nitro-, Cyano-, Hydroxyl-, Amino-, (C₁-C₆) -Alkylamino- oder Di- (C₁-C₆)-alkylamino-, Heterocycloalkyl-, Aryl-, Aryl-(C₁-C₆)-alkylen-, Heteroaryl-, Aryl-O- oder -C(O)-(C₁-C₆) -Alkylgruppen ausgewählte Gruppen substituiert sein kann, wobei die Heterocycloalkylgruppe gegebenenfalls durch -C(O)O(CH₃)₃ substituiert ist; steht;
n für 1 oder 2 steht;
m für 1 oder 2 steht;
o für 1 oder 2 steht;
in Basen- oder Säureadditionssalzform sowie in Hydrat- oder Solvatform.

3. Verbindung der Formel (I) nach Anspruch 1 oder 2, **dadurch gekennzeichnet, dass**
R für
ein Halogenatom
oder eine Hydroxylgruppe
oder eine Heterocycloalkylgruppe oder eine Arylgruppe oder eine Heteroarylgruppe; wobei diese Gruppe gegebenenfalls durch eine oder mehrere unter Halogenatomen und (C₁-C₆) -Alkyl-, (C₁-C₆)-Alkoxy-, (C₁-C₆) -Fluoralkyl-, (C₁-C₆) -Fluaralkoxy-, Di-(C₁-C₆)-alkylamino-, Heterocycloalkyl-, Aryl-, Aryl-(C₁-C₆)-alkylen-, Heteroaryl-, Aryl-O- oder -C(O)-(C₁-C₆)-Alkylgruppen ausgewählte Gruppen substituiert sein kann, wobei die Heterocycloalkylgruppe gegebenenfalls durch -C(O)O(CH₃)₃ substituiert ist;
steht;
n für 1 oder 2 sieht;
m für 1 oder 2 steht;
o für 1 steht;
in Basen- oder Säureadditionssalzform sowie in Hydrat- oder Solvatform.

4. Verbindung der Formel (I) nach einem der Ansprüche 1 bis 3, **dadurch gekennzeichnet, dass** n und m und o = 1; in Basen- oder Säureadditionssalzform sowie in Hydrat- oder Solvatform.

5. Verbindung der Formel (I) nach einem der Ansprüche 1 bis 3, **dadurch gekennzeichnet, dass** n und m =2 und o = 1; in Basen- oder Säureadditionssalzform sowie in Hydrat- oder Solvatform.

6. Arzneimittel, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure oder auch ein Hydrat oder ein Solvat enthält.

7. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Hilfsstoff enthält.

8. Verwendung einer Verbindung der Formel (I) nach einem der Ansprüche 1 bis 5 zur Herstellung eines Arzneimittels zur Behandlung und Prävention von kognitiven Störungen; Aufmerksamkeitsstörungen; Störungen der exekutiven Funktionen in Verbindung mit Alzheimer-Krankheit, pathologischer oder normaler Alterung, Parkinson-Syndrom, Trisomie 21, psychiatrischen Pathologien, Korsakoff-Alkoholsyndrom, vaskulären Demenzen oder Schädeltraumen; motorischen Störungen, die bei Parkinson-Krankheit oder anderen neurologischen Erkrankungen beobachtet werden, oder anatomisch-histopathologischen Störungen in Verbindung mit den oben aufgeführten neurodegenerativen Erkrankungen; oder multipler Sklerose.

9. Verwendung einer Verbindung der Formel (I) nach einem der Ansprüche 1 bis 5 zur Herstellung eines Arzneimittels zur Behandlung und Prävention von Schlaganfällen, zerebralen hypoxischen Episoden oder psychiatrischen Pathologien.

10. Verwendung einer Verbindung der Formel (I) nach einem der Ansprüche 1 bis 5 zur Herstellung eines Arzneimittels zur Prävention von durch Entzug von Tabak, Alkohol oder verschiedenen abhängigmachenden Substanzen verursachten Symptomen.

11. Verwendung einer Verbindung der Formel (I) nach einem der Ansprüche 1 bis 5 zur Herstellung eines Arzneimittels zur Behandlung von Schmerzen.

12. Verwendung einer Verbindung der Formel (I) nach einem der Ansprüche 1 bis 5 zur Herstellung eines Arzneimittels zur Behandlung von Ischämie der unteren Gliedmaßen, Arteritis obliterans der unteren Gliedmaßen, Herzischämie, Myokardinfarkt, Herzinsuffizienz, Hautvernarbungsdefizit bei Diabetespatienten, Ulcus varicosum bei Veneninsuffizienz oder septischem Schock.

13. Verwendung einer Verbindung der Formel (I) nach einem der Ansprüche 1 bis 5 zur Herstellung eines Arzneimittels zur Behandlung von Entzündungsprozessen.

14. Verwendung einer Verbindung der Formel (I) nach einem der Ansprüche 1 bis 5 zur Herstellung eines Arzneimittels zur Behandlung von dermatologischen Pathologien, Asthma oder Colitis ulcerosa.
